(19) Europäisches Patentamt / European Patent Office / Office européen des brevets

(11) **EP 3 597 175 A1**

(12) **EUROPEAN PATENT APPLICATION**
published in accordance with Art. 153(4) EPC

(43) Date of publication:
**22.01.2020 Bulletin 2020/04**

(21) Application number: **18768646.4**

(22) Date of filing: **15.03.2018**

(51) Int Cl.:
*A61K 8/73* (2006.01)   *A23L 33/10* (2016.01)
*A23L 33/16* (2016.01)   *A23L 33/18* (2016.01)
*A61K 8/21* (2006.01)   *A61K 8/365* (2006.01)
*A61K 8/64* (2006.01)   *A61K 31/191* (2006.01)
*A61K 31/7024* (2006.01)   *A61K 33/16* (2006.01)
*A61K 38/04* (2006.01)   *A61K 38/16* (2006.01)
*A61P 1/02* (2006.01)   *A61Q 11/00* (2006.01)

(86) International application number:
**PCT/JP2018/010217**

(87) International publication number:
**WO 2018/168997 (20.09.2018 Gazette 2018/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.03.2017   JP 2017051816**

(71) Applicant: **Ezaki Glico Co., Ltd.**
**Osaka-shi**
**Osaka 555-8502 (JP)**

(72) Inventors:
• **TANAKA, Tomoko**
  **Osaka-shi**
  **Osaka 555-8502 (JP)**
• **KOBAYASHI, Takatsugu**
  **Osaka-shi**
  **Osaka 555-8502 (JP)**
• **ASAKUMA, Hiroki**
  **Osaka-shi**
  **Osaka 555-8502 (JP)**

(74) Representative: **Uexküll & Stolberg**
**Partnerschaft von**
**Patent- und Rechtsanwälten mbB**
**Beselerstraße 4**
**22607 Hamburg (DE)**

(54) **ORAL COMPOSITION CAPABLE OF PROMOTING TEETH REMINERALIZATION**

(57)    The purpose of the present invention is to provide an oral composition that possesses an excellent teeth remineralization promoting effect and that is capable of effectively promoting teeth remineralization even when retained in the oral cavity for a short period of time. An oral composition in which (A) a calcium salt of organic acid, (B) a fluoride, and (C) a basic peptide and/or a basic protein in which 60% or more of the constituent amino acid residues are basic amino acid residues are added in combination is capable of dramatically promoting teeth remineralization as a result of a synergetic effect of these components and is capable of effectively promoting teeth remineralization even when retained in the oral cavity for a short period of time.

EP 3 597 175 A1

**Description**

TECHNICAL FIELD

The present invention relates to an oral composition which can promote tooth remineralization and is useful for anti-caries application.

BACKGROUND ART

The tooth is formed to contain hydroxyapatite ($Ca_{10}(PO_4)_6(OH)_2$) composed of calcium, phosphoric acid, and hydroxyl groups as a main component. The surface of tooth is covered with enamel having a dense structure in which aggregates of hexagonal columnar crystals of hydroxyapatite called enamel rod are aligned in a certain direction. This structure makes the tooth have a hardness comparable to that of quartz and the tooth is thus the hardest tissue in the living body. However, hydroxyapatite has the property of being chemically attacked by acids, and there is a possibility that enamel dissolves and calcium and phosphoric acid are eluted when the pH is equal to or less than the critical pH (usually pH 5.5).

When humans ingest carbohydrates, cariogenic bacteria in the oral cavity such as Streptococcus mutans assimilate the carbohydrates to produce acids. In addition, an insoluble glucan having an $\alpha$ (1 → 3) bond is formed from sucrose by the action of glucosyltransferase of these bacteria to form a biofilm. This is a viscid lump of bacteria, plaque (dental plaque). The enamel dissolves and the tooth is in a demineralized state when carbohydrate are continuously supplied to the plaque and acids are continuously produced. At this time, in the enamel, the hydroxyapatite density in the range of depth up to about 200 $\mu$m decreases while leaving the surface and a hollow structure is formed (subsurface demineralization). Furthermore, as the corrosion by acids proceeds, the surface collapses and the enamel surface is finally in a state of being perforated. At present, the tooth condition is called caries (carious cavity formed caries) for the first time in this state and is regarded as a stage in need of surgical treatment. Meanwhile, in the state of subsurface demineralization (early caries lesion, subsurface lesion), it is known that the tooth condition is reversibly recovered by the action of saliva without being subjected to surgical treatment. This is called remineralization. Hence, if demineralization and remineralization in the oral cavity are well balanced and remineralization rapidly occurs in the initial tooth decay state, the initial tooth decay does not proceed to tooth decay in need of dental treatment but can be recovered to the original healthy state.

Tooth remineralization by saliva is performed as calcium and phosphoric acid contained in the saliva are supplied to the subsurface demineralized site. However, the ratio (Ca/P molar ratio) of calcium to phosphorus in stimulated saliva is 0.4, and calcium is thus in short supply in the stimulated saliva since the Ca/P molar ratio in hydroxyapatite which is a constituent of tooth is 1.67. For this reason, when the oral cavity tends to be demineralized by the eating habits, hygiene habits and the like, remineralization to overcome demineralization is not attained only by supply of calcium and phosphoric acid from saliva and tooth decay gradually proceeds.

It is known that it is effective to supply calcium to saliva so that the Ca/P molar ratio in the saliva approaches the Ca/P molar ratio in hydroxyapatite for the promotion of tooth remineralization. Heretherto, various oral compositions have been proposed which are for remineralizing the tooth and contain water-soluble calcium. For example, Patent Documents 1 and 2 disclose that an oral composition containing calcium phosphate can promote tooth remineralization. In addition, Patent Document 3 discloses that calcium salt of phosphorylated saccharide has the action of suppressing the pH drop due to the acids produced by the bacteria in the cavity and the action of suppressing the formation of insoluble glucan produced by bacteria in the oral cavity in addition to the action of promoting tooth remineralization and can be used in a food and drink composition for anti-caries.

Meanwhile, fluorine is widely used to maintain the tooth in a healthy state in the dental field. It is known that the apatite becomes hard and the hardness of tooth can be ameliorated when a part or all of the hydroxyl groups in hydroxyapatite is fluorinated. It is known that a calcium fluoride-like substance is formed on the tooth surface and covers the tooth surface and an effect of resisting acids is attained when a fluoride, particularly a fluoride at a high concentration acts. However, this covering blocks the supply of calcium and phosphoric acid from saliva to the tooth and inhibits further remineralization in some cases. In addition, it is also known that fluoroapatite produced by fluorination of hydroxyapatite has a lower critical pH than hydroxyapatite and thus can impart acid resistance to the tooth itself. Hence, oral compositions having both the remineralization effect by a water-soluble calcium salt and the tooth quality ameliorating effect by fluorine have also been hitherto reported. Specifically, Patent Document 4 discloses that food which contains a calcium salt of phosphorylated saccharide, a fluoride, and polyphenol and is formulated so that these components have predetermined concentrations in saliva in the oral cavity can have both a high remineralization effect and a tooth quality ameliorating effect without causing precipitation and covering of calcium fluoride.

In addition, it is also known that basic amino acids such as lysine and arginine, basic peptides, basic proteins and the like can be used in the application of tooth restoration. For example, Patent Document 5 discloses that ε-poly-lysine has an anti-cariogenic action and an anti-periodontal disease action. In addition, Patent Document 6 discloses that an oral composition containing a basic peptide, a fluorine compound, and a sugar alcohol suppresses the pH drop due to the acid production by bacteria in the oral cavity and is effective for caries prevention. In addition, Patent Document 7 discloses an oral care composition containing a basic amino acid, a fraction of particles having a d50 of less than about 5 $\mu$m, which constitutes at least about 5% by weight of the formulation, and a soluble fluoride salt and discloses that the combination of a fluoride and a basic amino acid promotes remineralization. However, it has not been hitherto reported that basic peptides and basic proteins have an action of promoting tooth remineralization.

PRIOR ART DOCUMENTS

PATENT DOCUMENTS

Patent Document 1: Japanese Patent Laid-open Publication No. 10-310513
Patent Document 2: Japanese Patent Laid-open Publication No. 11-228369
Patent Document 3: Japanese Patent Laid-open Publication No. 2002-325557
Patent Document 4: International Publication No. 2010/61932
Patent Document 5: Japanese Patent Laid-open Publication No. 5-310544
Patent Document 6: Japanese Patent Laid-open Publication No. 2002-255773
Patent Document 7: Japanese Patent Laid-open Publication No. 2011-511066

SUMMARY OF THE INVENTION

PROBLEMS TO BE SOLVED BY THE INVENTION

There is room for further amelioration in the conventional oral compositions which promote tooth remineralization since the effect thereof is insufficient or it is required to retain the oral composition in the oral cavity for a long time for effect exertion. In addition, in recent years, the required performance for oral composition has been further improved in response to rising health awareness of consumers. Under such circumstances, there is a strong demand for the development of an oral composition acknowledged to have an effect of promoting tooth remineralization only by being retained in the oral cavity for a short time.

Hence, an object of the present invention is to provide an oral composition which has an excellent tooth remineralization promoting effect and can effectively promote tooth remineralization only by being retained in the oral cavity for a short time.

MEANS FOR SOLVING THE PROBLEM

The present inventors have intensively conducted investigations to solve the above-mentioned problems and, as a result, have been found out that a fluoride, a basic peptide, and a basic protein do not singly have an action of promoting tooth remineralization but an oral composition containing (A) a calcium salt of organic acid, (B) a fluoride, and (C) a basic peptide and/or a basic protein in which 60% or more of constituent amino acid residues is a basic amino acid residue can dramatically promote tooth remineralization by the synergistic effect of these components and can effectively promote tooth remineralization only by being retained in the oral cavity for a short time. The present invention has been completed by further investigations based on such findings.

In other words, the present invention provides the invention of aspects listed below.

Item 1. An oral composition containing (A) a calcium salt of organic acid, (B) a fluoride, and (C) a basic peptide and/or a basic protein in which 60% or more of constituent amino acid residues is a basic amino acid residue.

Item 2. The oral composition according to item 1, in which the component (A) is at least one selected from the group consisting of calcium salt of phosphorylated saccharide, calcium lactate, and calcium gluconate.

Item 3. The oral composition according to item 1 or 2, in which the component (A) is calcium salt of phosphorylated saccharide.

Item 4. The oral composition according to item 3, in which a saccharide moiety in the calcium salt of phosphorylated saccharide is glucan or reduced glucan. Item 5. The oral composition according to item 3 or 4, in which a degree of polymerization of a saccharide moiety in the calcium salt of phosphorylated saccharide is 2 to 10.

Item 6. The oral composition according to item 3 or 4, in which the number of phosphoric acid per one molecule is 1 or 2 in the calcium salt of phosphorylated saccharide.

Item 7. The oral composition according to any one of items 1 to 6, in which the component (C) is at least one selected from the group consisting of ε-poly-lysine, α-poly-lysine, and protamine.

Item 8. The oral composition according to any one of items 1 to 7, which is used in a tooth remineralization promoting application.

Item 9. The oral composition according to any one of items 1 to 8, which is food and drink.

Item 10. The oral composition according to any one of items 1 to 8, which is an oral care product.

Item 11. The oral composition according to any one of items 1 to 8, which is a pharmaceutical.

Item 12. Use of a composition containing (A) a calcium salt of organic acid, (B) a fluoride, and (C) a basic peptide and/or a basic protein in which 60% or more of constituent amino acid residues is a basic amino acid residue for the manufacture of an oral composition.

Items 13. The use according to item 12, in which the oral composition is an oral composition used for promotion of tooth remineralization.

Item 14. A method for promoting tooth remineralization, which includes orally administering or ingesting an oral composition containing (A) a calcium salt of organic acid, (B) a fluoride, and (C) a basic peptide and/or a basic protein in which 60% or more of constituent amino acid residues is a basic amino acid residue to a person in need of promotion of tooth remineralization.

ADVANTAGES OF THE INVENTION

According to the oral composition of the present invention, it is possible to dramatically promote tooth remineralization by a synergetic effect of (A) a calcium salt of organic acid, (B) a fluoride, and (C) a basic peptide and/or a basic protein in which 60% or more of the constituent amino acid residues are basic amino acid residues, and the oral composition can be thus suitably used in the anti-caries applications such as prevention of caries and amelioration of initial caries.

Furthermore, the oral composition of the present invention can effectively promote tooth remineralization only by being retained in the oral cavity for a short time and can thus effectively promote tooth remineralization even when the retention time in the oral cavity is shortened or the oral composition is provided as food (for example, tablet) having a relatively short retention time in the oral cavity.

In addition, calcium salt of phosphorylated saccharide among calcium salts of organic acid is relatively expensive but the oral composition of the present invention has a remarkably excellent tooth remineralization promoting effect and thus a sufficient effect can be attained even when low cost is achieved by decreasing the amount of calcium salt of phosphorylated saccharide used.

BRIEF DESCRIPTION OF THE DRAWINGS

Fig. 1 illustrates the results attained by measuring the mineral distribution (mineral profile) for every depth of enamel tooth pieces before and after being treated with remineralization treatment liquids of Example 1 and Comparative Example 6 in Test Example 1.

Fig. 2 illustrates the results attained by measuring the mineral distribution (mineral profile) for every depth of an enamel tooth piece in an in situ test to ingest a tablet of Example 11 in Test Example 2.

EMBODIMENTS OF THE INVENTION

The oral composition of the present invention contains a calcium salt of organic acid (hereinafter referred to as component (A) in some cases), a fluoride (hereinafter referred to as component (B) in some cases), and a basic peptide and/or a basic protein in which 60% or more of constituent amino acid residues is a basic amino acid residue (hereinafter referred to as component (C) in some cases) Hereinafter, the oral composition of the present invention will be described in detail.

Definition

In the present invention, "oral composition" refers to a composition to be applied to the oral cavity and includes food and drink, oral care products, and pharmaceuticals. In addition, "food and drink" is a concept including both foods and beverages. An "oral care product" is a product which is applied to the oral cavity but is not swallowed.

(A) Water-soluble calcium salt of organic acid

[Kind of component (A)]

The oral composition of the present invention contains a calcium salt of organic acid as a calcium source required for tooth remineralization. The calcium salt of organic acid singly exhibits tooth remineralization promoting action. However, as the component (B) and the component (C) to be described later are used together with the calcium salt of organic acid, the tooth remineralization promoting action of the oral composition of the present invention can be dramatically improved by the synergetic effect of these components.

The kind of calcium salt of organic acid used in the present invention is not particularly limited as long as the calcium salt of organic acid is applied to the oral cavity without causing any safety problems or is edible, but it is preferable that the calcium salt of organic acid is soluble in water so that calcium ions can be supplied to saliva in the oral cavity.

Specific examples of the calcium salt of organic acid used in the present invention include calcium salt of phosphorylated saccharide, calcium lactate, calcium gluconate, glucose-1-phosphate calcium, calcium acetate, calcium citrate, calcium succinate, calcium glutamate, calcium lactobionate, calcium malate, calcium formate, calcium benzoate, calcium isobutyrate, calcium propionate, calcium salicylate, calcium ascorbate, and casein phosphopeptide-amorphous calcium phosphate (CPP-ACP). These calcium salts of organic acid may be used singly or in combination of two or more kinds thereof.

Among these calcium salts of organic acid, calcium salt of phosphorylated saccharide, calcium lactate, and calcium gluconate are preferable and calcium salt of phosphorylated saccharide is still more preferable from the viewpoint of still further improving the tooth remineralization promoting action. Incidentally, the details of phosphorylated calcium salt to be suitably used as a calcium salt of organic acid in the present invention will be described later.

[Calcium salt of phosphorylated saccharide suitably used as component (A)]

"Calcium salt of phosphorylated saccharide" is a calcium salt of a saccharide having at least one phosphate group in the molecule.

The degree of polymerization (number of monosaccharides) of the saccharide moiety in the calcium salt of phosphorylated saccharide used in the present invention is not particularly limited but is, for example, 2 to 100, preferably 2 to 90, still more preferably 2 to 80, and particularly preferably 2 to 70, 2 to 60, 2 to 50, or 2 to 40. Particularly suitable examples of the degree of polymerization include 2 to 30 or 2 to 20. In particular, the calcium salt of phosphorylated saccharide used in the present invention is preferably a phosphorylated oligosaccharide calcium salt. The degree of polymerization of the saccharide moiety in the phosphorylated oligosaccharide calcium salt is specifically 2 to 10, 2 to 9, 2 to 8, 2 to 7, or 2 to 6, particularly preferably 2 to 5, and most preferably 3 to 5.

The kind of saccharide moiety constituting the calcium salt of phosphorylated saccharide used in the present invention is not particularly limited, but examples thereof include glucan, reduced glucan, xyloglucan, fructan, mannan, dextran, agar, cyclodextrin, fucoidan, gellan gum, locust bean gum, guar gum, tamarind gum, and xanthan gum. Among these, glucan or reduced glucan is preferable. Here, reduced glucan refers to one in which the aldehyde at the reducing end of glucan is reduced to an alcohol. Reduced glucan can be obtained, for example, by hydrogenating glucan and reducing the aldehyde to an alcohol. The glucan or reduced glucan constituting the saccharide moiety may have a linear structure in which glucose is linked by an $\alpha$-1,4 bond or a branched structure by $\alpha$-1,6 in the main chain in which glucose is linked by an $\alpha$-1,4 bond. As the glucan or reduced glucan constituting the saccharide moiety, a linear structure linked by an $\alpha$-1,4 bond is preferably mentioned.

In the calcium salt of phosphorylated saccharide, the phosphate group is bonded to the hydroxyl group in the saccharide by a phosphate ester bond. In the calcium salt of phosphorylated saccharide used in the present invention, the binding site of the phosphate group is not particularly limited, the phosphate group only needs to be bonded to at least one of hydroxyl groups not having glycosidic bond in the saccharide, but an aspect is preferable in which a phosphate group is bonded to at least either of a hydroxyl group present at the 3-position or a hydroxyl group present at the 6-position in the saccharide.

In the calcium salt of phosphorylated saccharide used in the present invention, the number of phosphate groups per one molecule is not particularly limited but is, for example, 1 to 10, preferably 1 to 5, still more preferably 1 to 3, and particularly preferably 1 or 2.

Calcium salt of phosphorylated saccharide is an inorganic salt of phosphorylated saccharide in which calcium is ionically bonded to the phosphate group in the phosphorylated saccharide. In the calcium salt of phosphorylated saccharide used in the present invention, the number of calcium atoms per one molecule is not particularly limited, and a calcium atom may be bonded to all of the phosphate groups present in the phosphorylated saccharide or a calcium atom may be bonded to only a part of the phosphate groups. The number of calcium atoms per one molecule of calcium salt of phosphorylated saccharide is specifically 1 to 20, preferably 1 to 5, still more preferably 1 to 3, and particularly preferably 1 or 2.

The molecular weight of the calcium salt of phosphorylated saccharide used in the present invention is not particularly limited but is, for example, 400 to 1,000,000, preferably 500 to 100,000, and still more preferably 600 to 10,000. A more suitable range of the molecular weight is 700 to 9000, 700 to 8000, 700 to 7000, 700 to 6000, 700 to 5000, 700 to 4000, or 700 to 3000, particularly preferably 700 to 2000, and most preferably 700 to 1000.

In the oral composition of the present invention, calcium salt of phosphorylated saccharide having one kind of structure may be used singly and calcium salts of phosphorylated saccharide having two or more kinds of structures may be used in combination as the component (A).

The calcium salt of phosphorylated saccharide can be obtained by phosphorylating a known saccharide to obtain a phosphorylated saccharide in the form of an acid and then converting the phosphorylated saccharide in the form of an acid into a calcium salt. The method for manufacturing calcium salt of phosphorylated saccharide is disclosed in detail in Japanese Patent Laid-open Publication No. 8-104696, Japanese Patent Laid-open Publication No. 2002-325557 and the like, and calcium salt of phosphorylated saccharide can be obtained according to the methods described in these publications. In addition, calcium salt of phosphorylated saccharide is commercially available from Glico Co., Ltd., Oji Cornstarch Co., Ltd. and the like, and commercially available products can be procured and used. In addition, the method for detecting calcium salt of phosphorylated saccharide is also disclosed, for example, in Japanese Patent Laid-open Publication No. 2002-325557 and the like and is known.

[Content of component (A)]

The content of the component (A) in the oral composition of the present invention varies depending on the form of oral composition, the kind of the component (A) to be used and the like but only needs to be set so that the Ca/P molar ratio in saliva in the oral cavity when the oral composition is applied to the oral cavity is about 1.0 to 2.0 and preferably about 1.67 of the Ca/P molar ratio in hydroxyapatite.

As a suitable aspect of the content of the component (A) in the oral composition of the present invention, an amount proper to have a calcium concentration in saliva in the oral cavity of 1.5 to 20 mM when the oral composition is applied to the oral cavity is mentioned. The content of the component (A) in the oral composition of the present invention is set to an amount proper to have a calcium concentration in saliva in the oral cavity of preferably 1.5 to 20 mM, still more preferably 2 to 10 mM, and particularly preferably 3 to 8 mM from the viewpoint of still further improving the tooth remineralization promoting action. Here, the amount proper to have a calcium concentration in saliva in the oral cavity of X mM when the oral composition is applied to the oral cavity refers to an amount in which the calcium concentration in saliva in the oral cavity during eating, during use, after eating, or after use can reach X mM when the oral composition is eaten or used. The calcium concentration in saliva in the oral cavity when the oral composition is applied to the oral cavity can be determined by collecting saliva and measuring the calcium concentration in the saliva. Here, saliva is not pure saliva secreted from the oral gland but refers to a liquid which accumulates in the oral cavity during eating or using the oral composition or after eating or using the oral composition. Human saliva is secreted in an average of 1 mL for one minute, and thus by taking the amount of saliva secreted, the amount of the oral composition eaten or used per one time, and the eating time or use time of the oral composition into consideration, those skilled in the art can easily set the content of the component (A) so that the calcium concentration in saliva in the oral cavity satisfies the above range when the oral composition is present in the oral cavity.

The content of the component (A) in the oral composition of the present invention is, specifically, 0.001% to 20% by weight, preferably 0.01% to 5% by weight, and still more preferably 0.02% to 1% by weight in terms of calcium content. In the present invention, with regard to the content of the component (A), terms of calcium content refers to the content of the component (A) calculated in terms of the weight of calcium atoms contained in the calcium salt of organic acid contained.

More specifically, the following range is mentioned as the content of the component (A) for every form of the oral composition of the present invention. Case in which oral composition of present invention is in form of food: the content of the component (A) is 0.001% to 10% by weight, preferably 0.01% to 2% by weight, and still more preferably 0.02% to 1% by weight in terms of calcium content. In particular, the content of the component (A) is particularly preferably 0.05% to 0.5% by weight in terms of calcium content in the case of a tablet and the content of the component (A) is particularly preferably 0.05% to 0.5% by weight and most preferably 0.05% to 0.2% by weight in terms of calcium content in the case of gum. Case in which oral composition of present invention is in form of beverage: the content of the component (A) is 0.001% to 4% by weight, preferably 0.01% to 1% by weight, and still more preferably 0.02% to 0.8% by weight in terms of calcium content. Case in which oral composition of present invention is oral care product: the content of the component (A) is 0.001% to 20% by weight, preferably 0.01% to 5% by weight, and still more preferably 0.02% to 1% by weight in terms of calcium content.

(B) Fluoride

[Kind of component (B)]

The oral composition of the present invention contains a fluoride as a component which promotes tooth remineralization. It is known that a fluoride singly has an effect of promoting tooth remineralization in the presence of calcium and phosphoric acid. However, as the component (A) described above and the component (C) to be described later are used together with a fluoride, the tooth remineralization promoting action of the oral composition of the present invention can be dramatically improved. In addition, a fluoride ion is likely to react with a calcium ion and cause precipitation, but it is possible to suppress the occurrence of precipitation due to fluoride ions and calcium ions by supplying calcium ions in the form of calcium salt of phosphorylated saccharide (Japanese Patent Laid-open Publication No. 2002-325557).
The kind of fluoride used in the present invention is not particularly limited as long as the fluoride is applied to the oral cavity without causing any safety problems or is edible, but it is preferable that the fluoride is soluble in water so that fluoride ions can be supplied to saliva in the oral cavity.
Specific examples of the fluoride used in the present invention include sodium fluoride, potassium fluoride, monofluorophosphoric acid and salts thereof (for example, sodium monofluorophosphate), sodium silicofluoride, hydrosilicofluoric acid, calcium fluoride, strontium fluoride, cryolite, monofluoroacetic acid, tin fluoride, and ammonium fluoride. These fluoride salts may be used singly or in combination of two or more kinds thereof.
Among these fluorides, sodium monofluorophosphate and sodium fluoride are preferable and sodium fluoride is still more preferable from the viewpoint of still further improving the tooth remineralization promoting action.
It is known that fluorides are also contained in plant extracts such as tea extract, and plant extracts containing fluorides may be used as the fluoride in the present invention.

[Content of component (B)]

The content of the component (B) in the oral composition of the present invention only needs to be appropriately set according to the form of the oral composition, the kind of the component (B) to be used and the like but is preferably an amount proper to have a fluorine concentration in saliva in the oral cavity of 0.03 to 300 ppm when the oral composition is applied to the oral cavity. The content of the component (B) in the oral composition of the present invention is set to an amount proper to have a fluorine concentration in saliva in the oral cavity of preferably 0.03 to 300 ppm, still more preferably 0.1 to 200 ppm, and particularly preferably 0.3 to 100 ppm when the oral composition is applied to the oral cavity from the viewpoint of still further improving the tooth remineralization promoting action. Here, the amount proper to have a fluorine concentration in saliva in the oral cavity of X ppm when the oral composition is applied to the oral cavity refers to an amount in which the fluorine concentration in saliva in the oral cavity during eating, during use, after eating, or after use can reach X ppm when the oral composition is eaten or used. The concentration of the component (B) in saliva in the oral cavity when the oral composition is applied to the oral cavity can be determined by collecting saliva and measuring the calcium concentration in the saliva. Human saliva is secreted in an average of 1 mL for one minute, and thus by taking the amount of saliva secreted, the amount of the oral composition eaten or used per one time, and the eating time or use time of the oral composition into consideration, those skilled in the art can easily set the content of the component (B) so that the fluorine concentration in saliva in the oral cavity satisfies the above range when the oral composition is applied to the oral cavity.
The content of the component (B) in the oral composition of the present invention is specifically 0.03 to 20000 ppm, preferably 0.1 to 1500 ppm, and still more preferably 0.3 to 500 ppm in terms of fluorine content. In the present invention, with regard to the content of the component (A), terms of fluorine content refers to the content of the component (A) calculated in terms of the weight of fluorine atoms contained in the fluoride contained.
More specifically, the following range is mentioned as the content of the component (B) for every form of the oral composition of the present invention. Case in which oral composition of present invention is in form of food: the content of the component (B) is 0.03 to 300 ppm, preferably 0.1 to 150 ppm, and still more preferably 0.3 to 20 ppm in terms of fluorine content. In particular, the content of the component (B) is particularly preferably 0.3 to 5 ppm in terms of fluorine content in the case of a tablet and the content of the component (B) is particularly preferably 0.3 to 10 ppm in terms of fluorine content in the case of gum. Case in which oral composition of present invention is in form of beverage: the content of the component (B) is 0.03 to 300 ppm, preferably 0.1 to 150 ppm, and still more preferably 0.3 to 20 ppm in terms of fluorine content. Case in which oral composition of present invention is oral care product: the content of the component (B) is 0.03 to 20000 ppm, preferably 0.1 to 1500 ppm, and still more preferably 0.3 to 600 ppm in terms of fluorine content.
In addition, in the oral composition of the present invention, the ratio of the component (A) to the component (B) is not particularly limited and is determined according to the contents of the component (A) and the component (B) described above. For example, the total amount of the component (B) is 0.05 to 30 parts by weight, preferably 0.1 to 20 parts by weight, and still more preferably 0.3 to 10 parts by weight per 100 parts by weight of the total amount of the component (A).

(C) Basic peptide and/or basic protein

The oral composition of the present invention contains a basic peptide and/or a basic protein in which 60% or more of constituent amino acid residues is a basic amino acid residue as a component which promotes tooth remineralization. The basic peptide and basic protein singly do not have a tooth remineralization promoting action. However, as the component (A) and component (B) described above are used together with the basic peptide and/or basic protein, the tooth remineralization promoting action of the oral composition of the present invention can be dramatically improved.

In the present invention, "peptide" refers to a peptide having less than 50 constituent amino acid residues. In addition, in the present invention, "protein" refers to a polypeptide having 50 or more constituent amino acid residues. In other words, the basic peptide used in the present invention is a peptide in which 60% or more of the constituent amino acid residues are basic amino acid residues and the number of constituent amino acid residues is less than 50. In addition, the basic protein used in the present invention is a polypeptide in which 60% or more of the constituent amino acid residues are basic amino acid residues and the number of constituent amino acid residues is 50 or more.

In the basic peptide and/or basic protein used in the present invention, 60% or more of the constituent amino acid residues are basic amino acid residues. It is possible to dramatically improve the tooth remineralization promoting action by using a basic peptide and/or a basic protein having a high ratio of basic amino acid residues in this manner. Here, the basic amino acid is the total amount of amino acids having residues exhibiting basicity, and specific examples of the basic amino acid include lysine, arginine, histidine, and ornithine.

In the basic peptide and basic protein used in the present invention, 60% or more of the constituent amino acid residues only need to be basic amino acid residues, but the proportion of basic amino acid residues to the total number of constituent amino acid residues is preferably 65% to 100%, still more preferably 70% to 100%, and particularly preferably 80% to 100% from the viewpoint of still further improving the tooth remineralization promoting action.

As a suitable aspect of the basic peptide and basic protein used in the present invention, the total amount of arginine residues and lysine residues is 60% or more, preferably 65% to 100%, still more preferably 70% to 100%, and particularly preferably 80% to 100% with respect to the total amount of constituent amino acid residues. Here, the "total amount of arginine residues and lysine residues" indicates the total number of lysine residues in a case in which the basic peptide and basic protein do not contain arginine residues but contain lysine residues, indicates the total number of arginine residues in a case in which the basic peptide and basic protein do not contain lysine residues but contain arginine residues, and indicates the total number of lysine residues and arginine residues in a case in which the basic peptide and basic protein contain both lysine residues and arginine residues.

The total number of constituent amino acid residues of the basic peptide used in the present invention only needs to be less than 50 but is preferably 5 to 45, still more preferably 10 to 40, and particularly preferably 25 to 35. Specific examples of the basic peptide used in the present invention include ε-poly-lysine, α-poly-lysine, protamine, poly-arginine, and a hydrolysate of a basic protein to be described later. Incidentally, the salmon-derived protamine is a peptide having 31 constituent amino acid residues including 21 L-lysine residues and is a basic peptide suitably used in the present invention. These basic peptides may be used singly or in combination of two or more kinds thereof.

The total number of constituent amino acid residues of the basic protein used in the present invention only needs to be 50 or more but is preferably 50 to 1000, still more preferably 50 to 500, and particularly preferably 50 to 200. Specific examples of the basic protein used in the present invention include ε-poly-lysine, α-poly-lysine, and poly-arginine. These basic proteins may be used singly or in combination of two or more kinds thereof.

In the oral composition of the present invention, as the component (C), one kind may be selected from basic peptides and basic proteins and used singly or two or more kinds may be used in arbitrary combination.

Among these basic peptides and basic proteins, ε-poly-lysine (including both peptide and protein cases), α-poly-lysine (including both peptide and protein cases), and protamine are preferable, ε-poly-lysine having a total number of lysine residues of 5 to 45 is still more preferable, and ε-poly-lysine having a total number of lysine residues of 25 to 35 is particularly preferable.

The basic peptides and basic proteins used in the present invention may be any of those obtained from natural products, those obtained by microorganism culture, those manufactured using genetic engineering techniques, or those chemically synthesized.

[Content of component (C)]

The content of the component (C) in the oral composition of the present invention only needs to be appropriately set according to the form of the oral composition, the kind of the component (C) to be used and the like, but is preferably an amount proper to have a total amount of basic peptide and basic protein in saliva in the oral cavity of 5 to 1500 ppm when the oral composition is applied to the oral cavity. The content of the component (C) in the oral composition of the present invention is set to an amount proper to have a concentration of a total amount of basic peptide and basic protein in saliva in the oral cavity of preferably 5 to 1500 ppm, still more preferably 10 to 500 ppm, and particularly preferably 15 to 300 ppm when the oral composition is applied to the oral cavity from the viewpoint of still further improving the tooth remineralization promoting action. Here, the concentration of the total amount of basic peptide and basic protein indicates the concentration of the total amount of basic protein in a case in which a basic peptide is not used but a basic protein is used, indicates the concentration of the total amount of basic peptide in a case in which a basic protein is not used but a basic peptide is used, and indicates the concentration of basic peptide and basic protein in a case in which both a basic peptide and a basic protein are used. Here, the amount proper to have a concentration of the total amount of basic peptide and basic protein in saliva in the oral cavity of X ppm when the oral composition is applied to the oral cavity refers to an amount in which the concentration of the total amount of basic peptide and basic protein in saliva in the oral cavity during eating, during use, after eating, or after use can reach X ppm when the oral composition is eaten or used. The fluorine concentration in saliva in the oral cavity when the oral composition is applied to the oral cavity can be determined by collecting saliva and measuring the concentration of the total amount of basic peptide and basic protein in the saliva. Human saliva is secreted in an average of 1 mL for one minute, and thus by taking the amount of saliva secreted, the amount of the oral composition eaten or used per one time, and the eating time or use time of the oral composition into consideration, those skilled in the art can easily set the content of the component (C) so that the concentration of the total amount of basic peptide and basic protein in saliva in the oral cavity satisfies the above range when the oral composition is applied to the oral cavity.

The content of the component (C) in the oral composition of the present invention is specifically 5 to 15000 ppm, preferably 10 to 5000 ppm, and still more preferably 15 to 3000 ppm.

More specifically, the following range is mentioned as the content of the component (C) for every form of the oral composition of the present invention. Case in which oral composition of present invention is in form of food: the content of the component (C) is 1 to 1000 ppm, preferably 5 to 250 ppm, and still more preferably 15 to 125 ppm. In particular, the content of the component (C) is particularly preferably 30 to 125 ppm in the case of a tablet or gum. Case in which oral composition of present invention is in form of beverage: the content of the component (C) is 1 to 1000 ppm by weight, preferably 5 to 250 ppm, and still more preferably 15 to 125 ppm. Case in which oral composition of present invention is oral care product: the content of the component (C) is 5 to 15000 ppm, preferably 10 to 5000 ppm, and still more preferably 15 to 3000 ppm.

In addition, in the oral composition of the present invention, the ratio of the component (A) to the component (C) is not particularly limited and is determined according to the component (A) in terms of calcium content and the content of the component (C) described above. For example, the component (C) is contained in a total amount of 1 to 100 parts by weight, preferably 2 to 50 parts by weight, and still more preferably 4 to 20 parts by weight per 100 parts by weight of the amount of the component (A) in terms of calcium content.

• Other components

The oral composition of the present invention may contain components commonly used in the technical field in addition to the components (A) to (C) according to the form of the oral composition in a range in which the effects of the present invention are not impaired.

Examples of such components include polyphenols, phosphate compounds, vitamins, amino acids, cellulose, starch, gelling agents, gum bases, acidulants, flour, sodium chloride, sugar alcohol, dietary fibers, lactic acid bacteria, fats and oils, abrasives, preservatives, disinfectants, antibacterial agents, anti-inflammatory agents, glucosyltransferase (GTase) inhibitors, plaque inhibitors, hypersensitivity inhibitors, dental plaque preventive agents, adhesives, thickeners, excipients, lubricants, flavors, acidulants, sweeteners, high-intensity sweeteners, refreshing agents, brighteners, coloring matters, deodorants, emulsifiers, pH adjusters, and various other food materials. The content of these components only needs to be appropriately set to the content commonly adopted in the technical field according to the form of the oral composition, the kind of components to be used, and the like.

• Shape and form

The shape of the oral composition of the present invention is not particularly limited and may be any of a liquid, a solid, a semi-solid (gel, ointment, paste) or the like.

In addition, the oral composition of the present invention may be either edible or non-edible as long as it can be applied to the oral cavity and stay in the oral cavity for a certain time. Specific examples of the form of the oral composition of the invention include food and drink, oral care products, and pharmaceuticals. Incidentally, in the present invention, the oral care products in Japan include products classified as quasi drugs or cosmetics.

In a case in which the oral composition of the present invention is prepared in the form of food and drink, it may be an insurance functional food such as foods with nutrient function claims and foods for specified health uses in addition to general food and drink. In a case in which the oral composition of the present invention is prepared in the form of food, examples of specific aspects thereof include chewing gums; candies; tablet confectionery; composite beverages; semi-liquid food such as yogurt; baked confectionery such as biscuits, rice crackers, and cookies; Frozen confectionery such as ice cream; gel-like food such as gummies and jellies; snacks; noodles; edible films; and supplements such as tablets, granules, fine grains, powders, and capsules. In addition, in a case in which the oral composition of the present invention is prepared in the form of drink, examples of specific aspects thereof include nutrient drinks, carbonated drinks, and soft drinks. Among these food and drink, chewing gums, candies, tablet confectionery, gel-like food, edible films, and supplements are preferable from the viewpoint of efficiently promoting tooth remineralization by securing a sufficient retention time in the oral cavity to supply effective amounts of the components (A) to (C) to saliva at the time of eating.

In a case in which the oral composition of the present invention is prepared into an oral care product, examples of specific aspects thereof include liquid dentifrices, toothpastes, moisturizers, powder dentifrices, mouthwashes (mouthwashes), mouth rinses, gargling agents, mouth sprays, pastes for oral cavity, gum massage creams, artificial saliva, mouth moisturizers, toothbrushing wipes, and dental floss.

In a case in which the oral composition of the present invention is prepared into a pharmaceutical, examples of specific aspects thereof include troches, tablets, pills, powders, solutions, suspensions, emulsions, granules, capsules, and coatings.

• Preparation method

The oral composition of the present invention can be prepared according to the preparation method known in the technical field according to the form.

• Application and usage

The oral composition of the present invention has the action of effectively promoting the tooth remineralization and can thus be used as a tooth remineralization promoter. In addition, promotion of tooth remineralization is effective for anti-caries applications (prevention of caries, amelioration of initial caries, and the like), and the oral composition of the present invention can also be used as a cariostatic agent (preventive agent for caries, remedy for initial caries, or the like). In other words, the oral composition of the present invention is suitably used for those who are seeking promotion of tooth remineralization, specifically those in need of prevention of dental caries, those in need of prevention of dental loss, those who are prone to develop initial caries, those with initial caries, and the like. Incidentally, in the present invention, "initial caries" is also called initial tooth decay and refers to a demineralized lesion which is localized on the enamel surface of tooth and does not form a cavity with defect of the tooth substance.

The oral composition of the present invention only needs to be applied to the oral cavity by a usual method according to the form of the oral composition. For example, the oral composition only needs to be eaten by a usual method according to the kind of food and drink in the case of food and drink, the oral composition only needs to be used by a usual method according to the kind of oral care product in the case of oral care products, and the oral composition only needs to be administered by a usual method according to the kind of pharmaceutical in the case of pharmaceuticals.

The oral composition of the present invention promotes tooth remineralization by supplying the components (A) to (C) to saliva in the oral cavity and is thus desirably eaten, used, or administered in an aspect in which the oral composition of the present invention is retained in the oral cavity for a certain time so that the effective amounts of the components (A) to (C) are supplied to saliva. In the prior art, it has not been possible to promote sufficient tooth remineralization in the case of a form (for example, tablet) in which the retention time of the oral composition in the oral cavity is relatively short, but the oral composition of the present invention can effectively promote tooth remineralization even in the form in which the retention time of the oral composition in the oral cavity is relatively short.

Specifically, the time during which the oral composition of the present invention is retained in the oral cavity only needs to be appropriately set according to the form of the oral composition, the concentrations of the components (A) to (C), and the like but is, for example, 20 seconds or more, preferably 20 seconds to 1 hour, still more preferably 60 seconds to 20 minutes, and particularly preferably 2 minutes to 10 minutes.

With regard to the amount of the oral composition of the present invention applied per one time, an effective amount in which tooth remineralization can be promoted only needs to be appropriately set according to the form of the oral composition, the expected effects, and the like. For example, the amount of the oral composition applied only needs to be set to an amount so that the amount of the component (A) applied is 0.1 mg or more, preferably 0.5 to 50 mg, still more preferably 1 to 30 mg, and particularly preferably 1.5 to 20 mg per one time.

The application frequency of the oral composition of the present invention is not particularly limited, and only needs to be appropriately set according to the expected effects and the like, but only needs to be set to, for example, about 1 to 10 times and preferably about 1 to 5 times a day, every day, every other day, every two days, and 1 to 3 days every week.

EXAMPLES

Hereinafter, the present invention will be described in detail based on Examples and the like, but the present invention is not limited thereto.

Incidentally, in the following Test Examples, one manufactured by Oji Cornstarch Co., Ltd. was used as calcium salt of phosphorylated saccharide (POs-Ca; Phosphoryl oligosaccharides of calcium). The calcium salt of phosphorylated saccharide contains calcium at 5.0% by weight. In addition, the calcium salt of phosphorylated saccharide mainly contains those of which the saccharide moiety is an oligosaccharide in which 3 to 5 glucoses are linked by an $\alpha$-1,4 bond and slightly contains those which are oligosaccharides in which 7 glucoses are linked by an $\alpha$-1,4 bond. Moreover, in the calcium salt of phosphorylated saccharide, one phosphate group is bonded in a case in which the saccharide moiety is an oligosaccharide containing 3 to 5 glucoses and two phosphate groups are bonded in a case in which the saccharide moiety is an oligosaccharide containing 7 glucoses.

Test Example 1: Verification of tooth remineralization promoting effect

1. Test materials

(1) Water

Deionized water manufactured using MilliQ (Merck Millipore, US) was used as water.

(2) Reagents

As calcium chloride, calcium lactate, calcium gluconate, $\alpha$-poly-lysine, protamine, potassium chloride, potassium dihydrogen phosphate, potassium hydroxide, hydrochloric acid (1 N aqueous solution), calcium chloride, L-lactic acid, and sodium fluoride, special grade products manufactured by FUJIFILM Wako Pure Chemical Corporation were used. One manufactured by DOJINDO LABORATORIES was used as HEPES (4-(2-hydroxyethyl)-1-piperazineethanesulfonic acid). As $\varepsilon$-poly-lysine, food grade $\varepsilon$-poly-L-lysine (degree of polymerization of lysine: 25 to 35) manufactured by JNC Corporation was used. As methylcellulose, Methocel MC (manufactured by SIGMA) was used. As poly-arginine, one having a molecular weight of 5,000 to 15,000 (manufactured by SIGMA) was used. As poly-histidine, one having a molecular weight of 5,000 to 25,000 (manufactured by SIGMA) was used. As chitosan, arginine, and histidine, those manufactured by FUJIFILM Wako Pure Chemical Corporation were used.

(3) Calcium salt of phosphorylated saccharide aqueous solution (POs-Ca aqueous solution)

In 8 ml of water, 1.0 g of calcium salt of phosphorylated saccharide (POs-Ca) was dissolved, this was diluted in a measuring cylinder to 10 ml, the solution was allowed to pass through a 0.45 $\mu$m filter (Minisart, Surfactant-free cellulose acetate, Sartorius Stedim Biotech, US) using a 10 ml syringe (Terumo Corporation), thereby obtaining a POs-Ca aqueous solution containing POs-Ca at 10% by weight.

(4) Stock solution of each reagent

Potassium dihydrogenphosphate and calcium chloride were dissolved in water so as to be 100 mM, potassium chloride 2 M, potassium hydroxide 1 M, sodium fluoride 1000 ppm, and HEPES 200 mM. After preparation, all of these were allowed to pass through a 0.45 $\mu$m filter (Minisart, Surfactant-free cellulose acetate, Sartorius Stedim Biotech, US). These were used as stock solutions.

(5) Enamel tooth piece

An enamel block (10 mm $\times$ 10 mm) was cut out from the crown of a bovine incisor, and then this block was embedded in a resin (UNIFAST TRAD manufactured by GC Corporation). This block was polished with dampened abrasive paper to expose a fresh flat enamel surface. Approximately 1/3 region of the enamel surface was covered by being coated with nail varnish and protected from the subsequent demineralization treatment. This part is the healthy part as the control. Thereafter, the enamel block was placed on the bottom of a 150 ml cylindrical container, and 75 ml of 8% by weight methyl cellulose gel dissolved by heating was poured therein and left to solidify at room temperature for 30 minutes. A 0.1 M L-lactic acid buffer solution (adjusted to pH 4.7) which had passed through a 0.22 $\mu$m filter and

sterilized in advance was overlaid thereon, and the resultant was incubated at 37°C for 14 days to form an enamel initial caries, namely, subsurface demineralized lesion in 2/3 region which was not covered with nail varnish (ten Cate JM et al., Caries Res. 40, 400-407, 1996). The degree of demineralization was determined by a method using Transversal microradiography (TMR) to be described later and, as a result, the mineral loss (ML) was in a range of 2000 to 3500 volume %•$\mu$m.

(6) Artificial saliva

An artificial lacrimal fluid is usually a solution having the composition presented in Table 1. In the present test, a solution in which the kind of calcium source (calcium chloride) contained in the artificial lacrimal fluid was changed and not an acid but potassium hydroxide was used for pH adjustment was used as a base of artificial saliva.

[Table 1]

| Artificial saliva | |
| --- | --- |
| Component contained | Concentration |
| KCl | 100 mM |
| HCl | 0.5 mM |
| $KH_2PO_4$ | 3.6 mM |
| $CaCl_2$ | 1.5 mM |
| HEPES | 20 mM |
| Water | Remainder |
| pH | 6.5 |

2. Test method

A remineralization treatment liquid containing a calcium source, a fluoride, a basic peptide, and a basic amino acid at predetermined concentrations was prepared using the stock solution of each reagent, a calcium source (calcium chloride, calcium phosphorylated oligosaccharide, calcium lactate, or calcium gluconate), a fluoride (sodium fluoride), a basic peptide ($\varepsilon$-poly-lysine, $\alpha$-poly-lysine, poly-arginine, polyhistidine, or protamine), and a basic amino acid (arginine or histidine) as a base of artificial saliva. The compositions of the remineralization treatment liquids prepared are as presented in Tables 2 and 3. Incidentally, the Ca/P molar concentration ratio is adjusted to be 1.67 in each remineralization treatment liquid.
The enamel tooth pieces subjected to the formation of initial caries were immersed in 100 mL of each remineralization treatment liquid and allowed to stand at 37°C for 6 hours or 24 hours. Thereafter, the enamel tooth pieces were analyzed according to the following TMR method.

<TMR method (Transversal microradiography method)>

A thin parallel section was cut out from the enamel tooth piece using a water cooled diamond saw. This thin section was polished to be a parallel horizontal plane and the thickness was adjusted to 150 $\mu$m. This thin section of enamel tooth piece was radiographed for 13 minutes by Cu-K$\alpha$ X-ray (PW-3830, Philips, The Netherlands) generated at 20 kV and 20 mA using a high resolution plate, developed, and microscopically analyzed. At the time of radiography, an aluminum step wedge was simultaneously radiographed as a standard substance and used to create a calibration curve of calcium content. The image of the enamel surface layer portion observed under a microscope and the calibration curve attained from the image density of the step wedge were converted into data for analysis using the software fair TMR 2012 ver. 4.00.23 developed by Inspektor Research Systems BV (The Netherlands). Thereafter, a mineral profile was created from the digital image using the software fair (TMR2006 ver. 3.0.0.17) developed by Inspektor Research Systems BV (The Netherlands). Various mineral parameters (lesion depth Ld, mineral loss (ML), and topmost surface layer point (Vmax)) were calculated by automatic analysis by the default settings of the software. Furthermore, the mineral recovery rate by remineralization in a case in which the enamel tooth piece was immersed in each remineralization treatment liquid for 6 hours was determined as follows. (i) First, the mineral loss from the demineralized portion and remineralized portion was determined from the mineral profile according to the automatic analysis of the software, (ii) Thereafter, the mineral recovery rate (%) when the mineral loss from the demineralized portion was taken as 100% loss was calculated based on the following formula.

[Math. 1]

$$\text{Mineral recovery rate } (\%) = [\{(\text{mineral loss from demineralized portion}) - (\text{mineral loss from remineralized portion})\}/(\text{mineral loss from demineralized portion})] \times 100$$

3. Test results

The results attained are presented in Tables 2 to 4. As presented in Comparative Example 6, the recovery rate was 20.5% in the case of the combination of POs-Ca with 1 ppm fluoride and was thus only slightly improved as compared with the case of POs-Ca single (Comparative Example 1, recovery rate: 16.1%). In addition, in Comparative Example 1 and Comparative Example 4, almost no difference was found in the recovery rate , and it can be thus said that the remineralization promoting effect is insufficient only by the combination of POs-Ca with ε-poly-lysine. Meanwhile, as presented in Example 1, in a case in which POs-Ca, 1 ppm fluoride, and 25 ppm ε-poly-lysine were combined, the recovery rate was 42.3% to be significantly improved, and it has been thus confirmed that remineralization is synergistically promoted by combining POs-Ca, a fluoride, and ε-poly-lysine.

In addition, it has also been confirmed from the comparison of Comparative Example 2 with Comparative Example 3 that remineralization is rather inhibited by the addition of poly-lysine when a calcium salt of inorganic acid (calcium chloride) is used as a calcium source. In the case of calcium chloride, although remineralization was ameliorated when the calcium concentration was increased as compared with a case in which ε-poly-lysine was not added, the stability of the solution itself was poor and precipitation gradually proceeded before remineralization. In fact, under the conditions of Comparative Example 7 (calcium chloride concentration: 6 mM), a remineralization rate was attained which was lower than that in Comparative Example 6 in which 1 ppm of fluoride was added to phosphorylated oligosaccharide calcium as a base. From this fact, in the case of using a calcium species which is likely to cause precipitation of phosphoric acid and calcium in the presence of phosphate ions (including monohydrogen phosphate and dihydrogen phosphate) such as calcium chloride, a remarkable remineralization promoting effect cannot be expected even this calcium species is combined with a fluoride and ε-poly-lysine. This tendency has also been acknowledged in a case in which calcium chloride, a fluoride, and polyarginine or polyhistidine are combined as presented in Comparative Examples 14 and 15. Furthermore, as presented in Examples 6 and 7, it has been confirmed that improvement in the recovery rate is acknowledged in the coexistence of a fluoride and ε-poly-lysine even when calcium lactate or calcium gluconate is used as a calcium source and improvement in the remineralization promoting effect is acknowledged when a calcium salt of organic acid as a calcium source is concurrently used with a fluoride and ε-poly-lysine.

Furthermore, as presented in Examples 8 to 10, it has been confirmed that the recovery rate is remarkably improved in the coexistence of POs-Ca and a fluoride even in a case in which α-poly-lysine, protamine or poly-arginine is used and improvement in the remineralization promoting effect is acknowledged in the coexistence of a calcium salt of organic acid and a fluoride even when a basic peptide or a basic polypeptide is used instead of ε-polyly-sine. Meanwhile, as presented in Comparative Examples 8 to 13, improvement in the recovery rate has not been acknowledged even in the coexistence of POs-Ca and a fluoride in a case in which arginine which is a basic amino acid or chitosan which is a basic polysaccharide is used as apparent from the comparison with Comparative Example 6.

Moreover, as presented in Examples 1, 4, and 5, it has been confirmed that the recovery rate increases concentration dependently when the fluoride concentration is increased to 100 ppm and a concentration in which a fluoride in saliva is 1 to 100 ppm is suitable for concurrent use with a calcium salt of organic acid and a basic peptide or basic polypeptide.

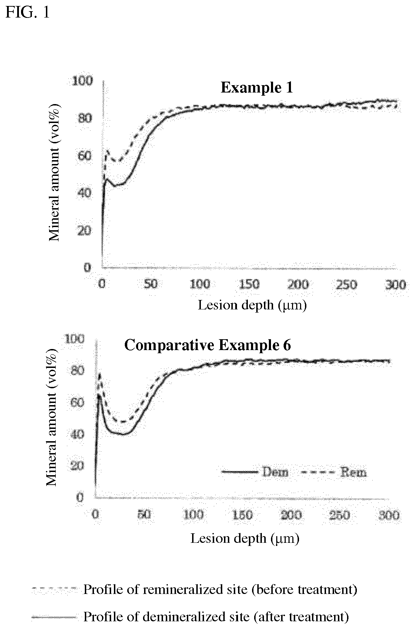

In addition, the results attained by measuring the mineral distribution (mineral profile) for every depth of enamel tooth pieces before and after being treated with the remineralization treatment liquids for Example 1 and Comparative Example 6 (after immersion for 24 hours) are illustrated in Fig. 1. As a result, only the demineralization depth was recovered in Comparative Example 6 in which only POs-Ca and ε-poly-lysine were combined, but recovery was observed in all of the demineralization depth, the mineral density in the topmost surface layer, and the minimum mineral density point in Example 1 in which POs-Ca, a fluoride, and ε-poly-lysine were combined. In other words, in Example 1, the density was recovered from the deep part, the thickness of the demineralized region decreased, and remineralization wholly proceeded. There is a possibility that penetration into deep portion is hindered when only the surface layer has a high density, and it can be thus said that it is more preferable for the recovery by remineralization of initial caries that remineralization proceeds from the deep layer as in Example 1.

Incidentally, under the condition in which the mineral recovery rate in the present test is calculated, the test is performed by immersing enamel tooth pieces in remineralization treatment liquids at 37°C for 6 hours, and it is a test system by which the remineralization effect can be evaluated in a short time. It can be said that the effect acknowledged in a case in which the oral composition is retained in the oral cavity for 3 minutes per one time and is used for 14 days at a frequency of 3 times a day can be evaluated by the condition of the present test.

[Table 2]

| | Comparative Example 1 | Comparative Example 2 | Comparative Example 3 | Comparative Example 4 | Comparative Example 5 | Comparative Example 6 | Comparative Example 7 | Example 1 | Example 2 | Example 3 | Example 4 | Example 5 |
|---|---|---|---|---|---|---|---|---|---|---|---|---|
| KCl | 100 mM | 100 mM | 100 mM | 100 mM | 100 mM | 100 mM | 100 mM | 100 mM | 100 mM | 100 mM | 100 mM | 100 mM |
| $KH_2PO_4$ | 3.6 mM | 3.6 mM | 3.6 mM | 3.6 mM | 3.6 mM | 3.6 mM | 3.6 mM | 3.6 mM | 3.6 mM | 3.6 mM | 3.6 mM | 3.6 mM |
| POs-Ca | 4800 ppm | - | - | 4800 ppm | 4800 ppm | 4800 ppm | - | 4800ppm | 4800 ppm | 4800 ppm | 4800 ppm | 4800 ppm |
| $CaCl_2$ | - | 1.5 mM | 1.5 mM | - | - | - | 6 mM | - | - | - | - | - |
| NaF | - | - | - | - | - | 1 ppm | 1 ppm | 1 ppm | 1 ppm | 1 ppm | 50 ppm | 100 ppm |
| ε-Poly-lysine | - | - | 25 ppm | 25 ppm | 250 ppm | - | 25 ppm | 25 ppm | 250 ppm | 1000 ppm | 25 ppm | 25 ppm |
| HEPES | 20 mM | 20 mM | 20 mM | 20 mM | 20 mM | 20 mM | 20 mM | 20 mM | 20 mM | 20 mM | 20 mM | 20 mM |
| KOH | Proper amount | Proper amount | Proper amount | Proper amount | Proper amount | Proper amount | Proper amount | Proper amount | Proper amount | Proper amount | Proper amount | Proper amount |
| Water | Remainder | Remainder | Remainder | Remainder | Remainder | Remainder | Remainder | Remainder | Remainder | Remainder | Remainder | Remainder |
| pH | 6.5 | 6.5 | 6.5 | 6.5 | 6.5 | 6.5 | 6.5 | 6.5 | 6.5 | 6.5 | 6.5 | 6.5 |
| Mineral recovery rate after immersion for 6 hours (%) | 16.1 | 13.7 | 0.7 | 15.2 | 19.7 | 20.5 | 16.8 | 42.3 | 35.0 | 25.8 | 45.0 | 51.3 |

EP 3 597 175 A1

[Table 3]

| | Comparative Example 8 | Comparative Example 9 | Comparative Example 10 | Comparative Example 11 | Comparative Example 12 | Comparative Example 13 | Example 6 | Example 7 | Example 8 | Example 9 |
|---|---|---|---|---|---|---|---|---|---|---|
| KCl | 100 mM | 100 mM | 100 mM | 100 mM | 100 mM | 100 mM | 100 mM | 100 mM | 100 mM | 100 mM |
| $KH_2PO_4$ | 3.6 mM | 3.6 mM | 3.6 mM | 3.6 mM | 3.6 mM | 3.6 mM | 3.6 mM | 3.6 mM | 3.6 mM | 3.6 mM |
| POs-Ca | 4800 ppm | 4800 ppm | 4800 ppm | 4800 ppm | 4800 ppm | 4800 ppm | - | - | 4800 ppm | 4800 ppm |
| Ca lactate | - | - | - | - | - | - | 6 mM | - | - | - |
| Ca gluconate | - | - | - | - | - | - | - | 6 mM | - | - |
| NaF | 1 ppm | - | 1 ppm | - | 1 ppm | 1 ppm | 1 ppm | 1 ppm | 1ppm | 1 ppm |
| ε-Poly-lysine | - | - | - | - | - | - | 25 ppm | 25 ppm | - | - |
| α-Poly-lysine | - | - | - | - | - | - | - | - | 25 ppm | - |
| Chitosan | 100 ppm | - | - | - | - | - | - | - | - | - |
| Protamine | - | - | - | - | - | - | - | - | - | 10000 ppm |
| Arginine | - | 25 ppm | 25 ppm | 40000 ppm | 20000 ppm | 40000 ppm | - | - | - | - |
| HEPES | 20 mM | 20 mM | 20 mM | 20 mM | 20 mM | 20 mM | 20 mM | 20 mM | 20 mM | 20 mM |
| KOH | Proper amount | Proper amount | Proper amount | Proper amount | Proper amount | Proper amount | Proper amount | Proper amount | Proper amount | Proper amount |
| Water | Remainder | Remainder | Remainder | Remainder | Remainder | Remainder | Remainder | Remainder | Remainder | Remainder |
| pH | 6.5 | 6.5 | 6.5 | 6.5 | 6.5 | 6.5 | 6.5 | 6.5 | 6.5 | 6.5 |
| Mineral recovery rate after immersion for 6 hours (%) | 23.3 | 7.8 | 8.9 | 7.3 | 10.0 | 11.4 | 29.2 | 23.1 | 45.7 | 35.9 |

EP 3 597 175 A1

16

[Table 4]

|  | Comparative Example 14 | Comparative Example 15 | Example 10 |
|---|---|---|---|
| KCl | 100 mM | 100 mM | 100 mM |
| $KH_2PO_4$ | 3.6 mM | 3.6 mM | 3.6 mM |
| $CaCl_2$ | 1.5 mM | 1.5 mM | - |
| POs-Ca | - | - | 4800 ppm |
| NaF | 1 ppm | 1 ppm | 1 ppm |
| Polyhistidine | 25 ppm | - | - |
| Polyarginine | - | 25 ppm | 25 ppm |
| HEPES | 20 mM | 20 mM | 20 mM |
| KOH | Proper amount | Proper amount | Proper amount |
| Water | Remainder | Remainder | Remainder |
| pH | 6.5 | 6.5 | 6.5 |
| Mineral recovery rate after immersion for 6 hours (%) | 6.8 | 6.7 | 35.9 |

Test Example 2: Verification of in situ tooth remineralization promoting effect

1. Test method

Tablets (0.83 g per tablet) having the composition presented in Table 5 were prepared according to a conventional method. The tablets of Example 11 were adjusted so that the calcium ion in saliva at the time of ingestion was about 6 mM, ε-poly-lysine about 25 ppm, and the fluoride concentration (derived from green tea extract) about 1 ppm. An intraoral device having a resin part provided with a recess to which the enamel block was attached, a metal mesh lid part which protected the enamel block, and a metal hook for hanging on the buccal side of the mandible tooth and holding the resin part from the root area on the back side of the front tooth to the lower part of the tongue tip was prepared (manufactured by Tokyo Medical And Dental University School For Dental Technicians). The intraoral device was worn to the lower jaw of the subject in a state in which an enamel block demineralized by the same method as in Test Example 1 was attached to the recess of the intraoral device and protected by the metal mesh lid. Thereafter, one tablet was ingested at a time, three times a day, for 14 days after every meal in a state in which the intraoral device was worn. The intraoral device was worn in the mouth of the subject from 10 minutes before ingestion to 10 minutes after ingestion every time the tablet was ingested, was removed from the lower jaw with the enamel block attached, lightly washed with water, and then stored in a state of not being dried for the time other than this. At the time point at which ingestion for 14 days was ended, the enamel tooth piece attached to the intraoral device was analyzed according to the TMR method described in Test Example 1.

[Table 5]

|  | Example 11 |
|---|---|
| Calcium salt of phosphorylated saccharide (POs-Ca) | 5.00 |
| Green tea powder (containing fluoride)[#1] | 1.50 |
| ε-Poly-lysine (purity: 50%)[#2] | 0.04 |
| Sorbitol | 86.00 |
| Sucrose fatty acid ester | 2.50 |
| Calcium stearate | 0.50 |
| Silicon oxide fine particles | 0.50 |

(continued)

|  | Example 11 |
|---|---|
| Flavor power | Proper amount |
| High-intensity sweetener[#3] | Proper amount |
| Sum (% by weight) | 100 |

#1 One (fluorine concentration: 1000 ppm) manufactured by Mitsui Norin Co., Ltd. was used as the green tea powder.
#2 Food grade $\varepsilon$-poly-L-lysine (degree of polymerization of lysine: 25 to 35) manufactured by JNC Corporation was used as $\varepsilon$-poly-lysine.
#3 A mixture of acesulfame potassium, an aspartame L-phenylalanine compound, and sucralose was used as the high-intensity sweetener.

## 2. Test results

The recovery rate of demineralization depth at the remineralized portion was 14.5%, and the recovery rate (%) of calcium at the remineralized portion was 27.5%. The ingestion time of tablet is shortened by about 1/3 to 1/5 as compared with that of gum, but the effect of remineralizing initial caries by the tablet of Example 11 has been acknowledged even when the ingestion time is short.

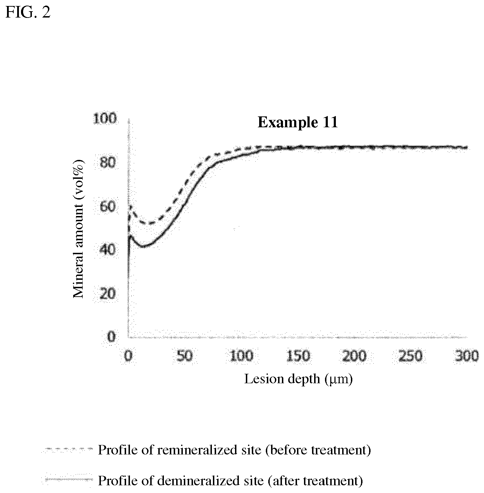

Moreover, the results attained by measuring the mineral distribution (mineral profile) for every depth of the enamel tooth piece in a case in which the tablet of Example 11 was ingested are illustrated in Fig. 2. From this result, it has been confirmed that tooth remineralization is remarkably promoted in a case in which a calcium salt of organic acid, a fluoride, and a basic peptide are concurrently used even in an in situ test since recovery of the lesion depth (Ld) and the topmost surface layer point (Vmax) is also observed in a case in which the tablet of Example 11 is ingested.

**Claims**

1. An oral composition comprising:

    (A) a calcium salt of organic acid;
    (B) a fluoride; and
    (C) a basic peptide and/or a basic protein in which 60% or more of constituent amino acid residues is a basic amino acid residue.

2. The oral composition according to claim 1, wherein the component (A) is at least one selected from the group consisting of calcium salt of phosphorylated saccharide, calcium lactate, and calcium gluconate.

3. The oral composition according to claim 1 or 2, wherein the component (A) is calcium salt of phosphorylated saccharide.

4. The oral composition according to claim 3, wherein a saccharide moiety in the calcium salt of phosphorylated saccharide is glucan or reduced glucan.

5. The oral composition according to claim 3 or 4, wherein a degree of polymerization of a saccharide moiety in the calcium salt of phosphorylated saccharide is 2 to 10.

6. The oral composition according to claim 3 or 4, wherein the number of phosphoric acid per one molecule is 1 or 2 in the calcium salt of phosphorylated saccharide.

7. The oral composition according to any one of claims 1 to 6, wherein the component (C) is at least one selected from the group consisting of $\varepsilon$-poly-lysine, $\alpha$-poly-lysine, and protamine.

8. The oral composition according to any one of claims 1 to 7, which is used in a tooth remineralization promoting application.

9. The oral composition according to any one of claims 1 to 8, which is food and drink.

10. The oral composition according to any one of claims 1 to 8, which is an oral care product.

11. The oral composition according to any one of claims 1 to 8, which is a pharmaceutical.

12. Use of a composition containing (A) a calcium salt of organic acid, (B) a fluoride, and (C) a basic peptide and/or a basic protein in which 60% or more of constituent amino acid residues is a basic amino acid residue for the manufacture of an oral composition.

13. The use according to claim 12, wherein the oral composition is an oral composition used for promotion of tooth remineralization.

14. A method for promoting tooth remineralization, which comprises orally administering or ingesting an oral composition containing (A) a calcium salt of organic acid, (B) a fluoride, and (C) a basic peptide and/or a basic protein in which 60% or more of constituent amino acid residues is a basic amino acid residue to a person in need of promotion of tooth remineralization.

FIG. 1

Profile of remineralized site (before treatment)

Profile of demineralized site (after treatment)

FIG. 2

Example 11

Mineral amount (vol%) vs Lesion depth (μm)

- - - - - Profile of remineralized site (before treatment)

————— Profile of demineralized site (after treatment)

**INTERNATIONAL SEARCH REPORT**

| International application No. |
| --- |
| PCT/JP2018/010217 |

**A. CLASSIFICATION OF SUBJECT MATTER**
Int.Cl. [see extra sheet]

According to International Patent Classification (IPC) or to both national classification and IPC

**B. FIELDS SEARCHED**

Minimum documentation searched (classification system followed by classification symbols)
Int.Cl. A61K8/73, A23L33/10, A23L33/16, A23L33/18, A61K8/21, A61K8/365, A61K8/64, A61K31/191, A61K31/7024, A61K33/16, A61K38/04, A61K38/16, A61P1/02, A61Q11/00

Documentation searched other than minimum documentation to the extent that such documents are included in the fields searched
Published examined utility model applications of Japan    1922-1996
Published unexamined utility model applications of Japan  1971-2018
Registered utility model specifications of Japan          1996-2018
Published registered utility model applications of Japan  1994-2018

Electronic data base consulted during the international search (name of data base and, where practicable, search terms used)
JSTPlus/JMEDPlus/JST7580(JDreamIII), CAplus/KOSMET/MEDLINE/BIOSIS(STN), Mintel GNPD

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2011-511091 A (COLGATE PALMOLIVE CO.) 07 April 2011, claim 1, 3-5, 8, 36, 38-41<br>& US 2010/0330002 A1, claim 1, 3-5, 8, 36, 38-41 & WO 2009/100260 A2 & EP 2249794 A2 | 1-2, 7-12<br>1-12 |
| X | JP 2002-255773 A (LION CORPORATION) 11 September 2002, claim 1, abstract, [0001], [0007], [0012], [0013], [0026], example 4<br>(Family: none) | 1, 7-12 |

☒ Further documents are listed in the continuation of Box C.   ☐ See patent family annex.

| | |
| --- | --- |
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>26.04.2018 | Date of mailing of the international search report<br>22.05.2018 |
| --- | --- |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2018/010217

**C (Continuation).   DOCUMENTS CONSIDERED TO BE RELEVANT**

| Category* | Citation of document, with indication, where appropriate, of the relevant passages | Relevant to claim No. |
|---|---|---|
| Y | WO 2010/061932 A1 (EZAKI GLICO CO., LTD.) 03 June 2010, paragraph [0001], claims 1, 6-8, 15, 19-21, 29<br>& US 2011/0256072 A1, paragraph [0001], claims 1, 6-8 & JP 5726533 B2 | 1-12 |
| Y | JP 2002-325556 A (EZAKI GLICO CO., LTD.) 12 November 2002, claims 1-16, paragraph [0001] (Family: none) | 1-12 |
| A | JP 2000-154127 A (LION CORPORATION) 06 June 2000, entire text (Family: none) | 1-12 |
| A | JP 06-298631 A (KAO CORPORATION) 25 October 1994, entire text (Family: none) | 1-12 |
| A | JP 2002-12536 A (LION CORPORATION) 15 January 2002, entire text (Family: none) | 1-12 |
| A | Tooth brushing gel for babies, ID1947152, Mintel GNPD [online], December 2012, [retrieved on 23 April 2018], Internet <http://www.portal.mintel.com> | 1-12 |

Form PCT/ISA/210 (continuation of second sheet) (January 2015)

**INTERNATIONAL SEARCH REPORT**

International application No.

PCT/JP2018/010217

(Continuation of Box No. A)

CLASSIFICATION OF SUBJECT MATTER
A61K8/73(2006.01)i, A23L33/10(2016.01)i, A23L33/16(2016.01)i,
A23L33/18(2016.01)i, A61K8/21(2006.01)i, A61K8/365(2006.01)i,
A61K8/64(2006.01)i, A61K31/191(2006.01)i, A61K31/7024(2006.01)i,
A61K33/16(2006.01)i, A61K38/04(2006.01)i, A61K38/16(2006.01)i,
A61P1/02(2006.01)i, A61Q11/00(2006.01)i

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10310513 A
- JP 11228369 A
- JP 2002325557 A
- WO 201061932 A
- JP 5310544 A
- JP 2002255773 A
- JP 2011511066 A
- JP 8104696 A

**Non-patent literature cited in the description**

- **TEN CATE JM et al.** *Caries Res.,* 1996, vol. 40, 400-407